(19) **Europäisches Patentamt**
**European Patent Office**
**Office européen des brevets**

(11) **EP 4 043 900 A1**

(12) **EUROPEAN PATENT APPLICATION**

(43) Date of publication:
**17.08.2022 Bulletin 2022/33**

(21) Application number: **21157287.0**

(22) Date of filing: **16.02.2021**

(51) International Patent Classification (IPC):
**G01R 33/28** $^{(2006.01)}$ **A61B 5/01** $^{(2006.01)}$

(52) Cooperative Patent Classification (CPC):
**G01R 33/288; A61B 5/01; A61B 5/015;**
**A61B 5/055; A61F 7/00; G01R 33/28;** G01R 33/283

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventor: **WEISS, Steffen**
**5656 AE Eindhoven (NL)**

(74) Representative: **Philips Intellectual Property &**
**Standards**
**High Tech Campus 52**
**5656 AG Eindhoven (NL)**

(54) **CONTROLLING PATIENT TEMPERATURE IN AN MR SCANNER BORE**

(57) A system is provided for controlling the temperature of a patient during an MR scan. A face skin temperature measurement is taken by non-contact thermal sensing. A patient temperature is estimated based on the skin temperature measurement and a cooling system is controlled in dependence on the estimated patient temperature.

FIG. 2

**Description**

FIELD OF THE INVENTION

This invention relates to controlling the temperature of a patient in an MR (magnetic resonance) scanner bore.

BACKGROUND OF THE INVENTION

Magnetic resonance imaging (MRI) is a medical imaging technique used in radiology to form pictures of the anatomy and the physiological processes of the body. MRI scanners use strong magnetic fields, magnetic field gradients, and radio waves to generate images of the organs in the body.

MRI is widely used in hospitals and clinics for medical diagnosis and staging and follow-up of disease without exposing the body to radiation. MRI is based on the ability of certain atomic nuclei to absorb radio frequency energy when placed in an external magnetic field. The resultant evolving spin polarization can induce an RF signal in a radio frequency coil and thereby be detected.

Hydrogen atoms are most often used to generate a macroscopic polarization that is detected by antennas close to the patient being examined. Hydrogen atoms are naturally abundant in humans and other biological organisms, particularly in water and fat. For this reason, most MRI scans essentially map the location of water and fat in the body.

Pulses of radio waves excite the nuclear spin energy transition, and magnetic field gradients localize the polarization in space. By varying the parameters of the pulse sequence, different contrasts may be generated between tissues based on the relaxation properties of the hydrogen atoms therein.

During scanning, there may be scan-induced RF heating which increases the ambient temperature. The clinical clothing or equipment worn may also result in an increase in temperature of the patient. For example, a patient may be wrapped in surface coils, with straps to fix them closely to the body.

The temperature of the patient may also vary due to nervousness or anxiety, and the change in patient physiological state, such as reduced physical activity or even sleep, and possible sedation, can cause changes in patient temperature.

Any change in body temperature during MR scanning induces inconvenience for the patient and should be avoided.

The body temperature of a patient may change during MR scanning for the various reasons outlined above. The influence of these different conditions varies in different situations, and their influence on the body temperature of the patient is hard to predict.

Most MR scanners are equipped with a fan that can ventilate the bore at several different fan speeds and respective cooling rates. However, staff are often so busy that patient convenience is not prioritized. It adds additional workload to staff to ask the patient (repeatedly) if the temperature level is convenient. Patients also typically do not know that the fan speed can be adjusted and therefore will not ask for a change in temperature when it is needed.

WO 2016/087376 discloses an MRI system in which multiple infrared thermometry sensors are provided around the body of the patient. Each sensor is associated with a loop antenna element, which are the potential hot spots of the surface coil that are prone to heating during scanning. The sensors measure surface temperatures of the subject. If a surface temperature is above a predefined temperature, an action is taken. This action may be to reduce the RF power supplied to a loop antenna element to reduce local heating. In another example, an air flow of an air cooling system can be increased.

This requires sensors to be attached to or worn by the patient which results in additional discomfort and anxiety.

SUMMARY OF THE INVENTION

The invention is defined by the claims.

According to examples in accordance with an aspect of the invention, there is provided a system for controlling the temperature of a patient during an MR scan, comprising:

a non-contact system for performing a skin temperature measurement of the face; and
a controller, configured to:

estimate an overall patient temperature based on the skin temperature measurement; and
provide a cooling system control signal for controlling a cooling system in dependence on the estimated patient temperature, for cooling the patient.

This system measures a skin temperature of the face of a patient, by a remote non-contact sensor, and then provides a cooling system control signal to automatically adjust a cooling system to keep the patient comfortable, for example to keep the temperature constant or within an allowed range. In this way, no input is needed from the patient, and the automated control allows clinicians to concentrate or more clinically important tasks.

The "overall" patient temperature estimate is intended to be a global temperature measurement, indicative of generally how hot or cold the patient is feeling. The system thus enables automatic control of the global patient temperature and hence related patient comfort.

The face is typically exposed during an MR scan so it provided a convenient area of the body for which non-contact temperature measurement, such as using an imaging procedure, may be performed.

The cooling system control signal is for example a fan speed control signal for controlling a fan speed, for providing ventilation to the patient. The system thus automatically adjusts a fan speed to keep the patient comfortable.

The controller is for example configured to determine an average temperature for a plurality of locations of the face. Thus, the non-contact skin temperature measurement may cover an area of the face (or the whole face) and an average is obtained to represent the body temperature of the patient.

The controller may be configured to derive a reference temperature within a first time period of the patient entering an MR scanner bore. This reference temperature may then be used as an indication of body temperature of the patient at the beginning of the scan, and the controller may control the fan speed to maintain the body temperature at this reference level, or within a defined range of temperatures compared to this reference.

The first time period is for example in the range 30 seconds to 5 minutes and represents the initial body temperature before any influence of the MR scan procedure is manifested.

The non-contact system for example comprises an infrared camera system or a laser infrared thermometer. An infrared camera system for example captures a video stream of a thermal camera of the face of the patient. Alternatively, (non-contact) laser thermometers may be built into the MR bore or even into a head coil used for neurofunctional examination. In all cases, the non-contact temperature measurement system does not require additional parts to be attached to or worn by the patient and it provides a measurement at the face of the patient.

The controller may be configured to provide a fan speed control signal for controlling a fan speed to maintain a constant estimated temperature using proportional-integral (PI) or proportional-integral-differential (PID) control.

The PI or PID control may use only the estimated temperature as input parameter. Thus the feedback system then controls the fan speed setting based on the estimated temperature, for example the deviation from a reference.

The PI or PID control may however use the estimated temperature as well as thermal absorption characteristics of the MR scan as inputs.

In particular, further variables such as the specific absorption rate (SAR) burden of the scan may be used to feed a more complex model that estimates the heating rate of the patient. This model is again used to adjust fan speed with the aim to keep the body temperature constant.

The temperature control system may further comprise a voice command system for receiving voice commands from the patient, and the controller is configured to adapt the fan speed control signal in dependence on the voice commands. These voice commands allow the patient to provide input, which may for example be adapted to alter the automated control, for example by changing a reference temperature.

The invention also provides a magnetic resonance imaging system, comprising:

an MR bore;
a cooling system for cooling a patient in the MR bore; and
the temperature control system as defined above.

The cooling system for example comprise a fan.

The invention also provides a computer implemented method for controlling the temperature of a patient during an MR scan, comprising:

performing a skin temperature measurement of the face using a non-contact system;
estimating an overall patient temperature based on the skin temperature measurement; and
controlling a cooling system in dependence on the estimated patient temperature for cooling the patient.

The cooling system may be a ventilator fan.

The method may comprise performing a skin temperature measurement of the face using an infrared camera system or a laser infrared thermometer. The method may comprise controlling a fan speed to maintain a constant estimated temperature using PI or PID control.

The invention also provides a computer program comprising computer program code means which is adapted, when said program is run on a computer, to implement the method as defined above.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Figure 1 shows in simplified schematic form an MRI system with a patient within the scanner bore;
Figure 2 shows an MR scanner equipped with a system for controlling the temperature of the patient during the MR scan;
Figure 3 shows a thermal camera image showing how minute temperature differences at different facial locations can be captured; and
Figure 4 shows a method for controlling the temperature of a patient during an MR scan.

DETAILED DESCRIPTION OF THE EMBODIMENTS

The invention will be described with reference to the Figures.
It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.
The invention provides a system for controlling the temperature of a patient during an MR scan. A face skin temperature measurement is taken by non-contact thermal sensing. A patient temperature is estimated based on the skin temperature measurement and a cooling system is controlled in dependence on the estimated patient temperature.

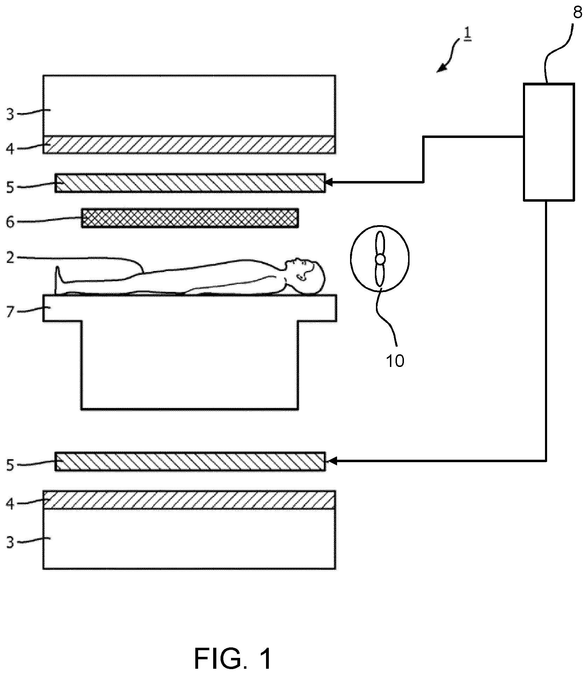

Figure 1 shows in simplified schematic form an MRI system (scanner) with a patient 2 within the scanner bore. The MRI system 1 is only shown with its most fundamental components. In this respect, the MRI system 1 comprises a gradient coil 4 within the bore of the superconducting magnet 3 as well as RF transmit coils 5 and a RF receiver coil 6.
Local coils are applied to the patient within the MR bore, and these local coils are referred to as the "patient coil". The patient coil may include multiple individual coil elements and typically functions as the RF receiver coil 6.
The RF transmit coils 5 emit RF pulses, which are supplied from a RF transmitter 8, and generate a radio frequency magnetic field within the bore of the superconducting magnet 3.
As is well known by one of ordinary skill in the art, by transmitting RF pulses which have an orthogonal polarization to the magnetic field generated by the superconducting magnet 3 and matching the Larmor frequency of the nucleons of interest, the spins of the nucleons can be excited and brought into phase, and a deflection of their net magnetization from the direction of the field of the superconducting magnet 3 is obtained so that a transversal component in relation to the longitudinal component of the net magnetization is generated.
After termination of the RF pulse, the relaxation process of the longitudinal and reversal components of the net magnetization begins until the net magnetization has returned to its equilibrium state. Magnetic resonance signals, which are generated by the precessing magnetization, are detected by means of the RF receiver coil 6, i.e. the patient coil.
The received magnetic resonance signals are time-based amplitude signals, which are further Fourier transformed to frequency-based magnetic resonance spectrum signals and further processed for generating a magnetic resonance image of the nucleons of interest.
The RF transmitter 8 comprises an RF amplifier for generating RF pulses and for forwarding these RF pulses to the RF transmit coil 5 of the MRI system 1. Further, the RF transmitter 8 typically comprises a capacitor bank which is coupled to the RF amplifier, for storing electric energy and for providing the RF amplifier with a current for generating the RF pulses. A mains power supply is coupled to the capacitor bank, for generating a charging current for charging the capacitor bank with electric energy.
A fan 10 is typically used to control the environmental conditions for the patient 2. The fan 10 can ventilate the bore at several different fan speeds and respective cooling rates. The fan is one example of a possible cooling system for cooling the patient.

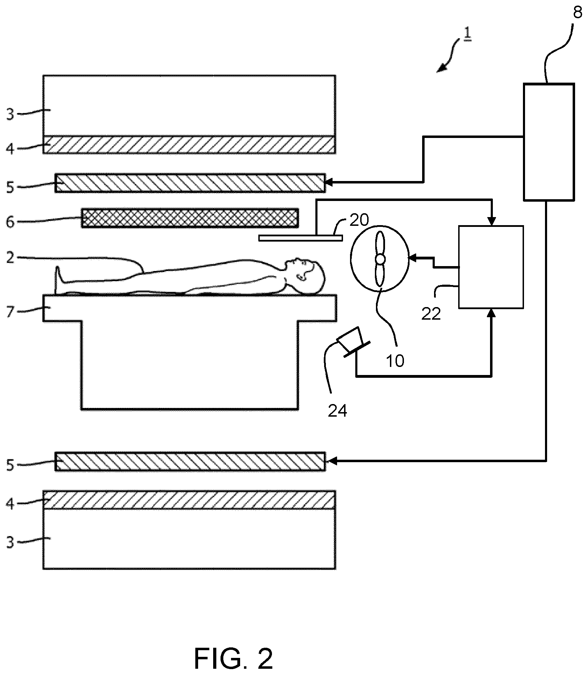

Figure 2 shows an MR scanner equipped with a system for controlling the temperature of the patient 2 during the MR scan.
The temperature control system comprises a non-contact system 20 for performing a skin temperature measurement, in particular of the face of the patient. A controller 22 estimates a patient temperature based on the skin temperature measurement.

In a simplest implementation, a single skin temperature measurement is obtained, and this is used as the estimate of the body temperature of the patient. Instead, there may be multiple temperature measurements, and a single estimated body temperature is derived from those multiple measurements, for example as an average.

The estimated body temperature is a surface body temperature rather than a core body temperature, and it is intended to indicate how warm or cold the patient feels, and is thus indicative of a global/general level of comfort rather than being indicative of any particular underlying physiological condition of the patient or any particular localized heating effect. Thus, the estimated body temperature is an indication of generally how hot or cold the patient feels.

In preferred arrangements, the skin temperature measurement is taken at areas which are exposed to the ambient surroundings, hence not in proximity to a patient coil, so that the measurement is not intended to capture local surface heating effects caused by the patient coil.

The controller 22 provides a fan speed control signal for controlling a speed of the fan 10 in dependence on the estimated patient temperature, for providing ventilation to the patient.

The system thus measures a skin temperature of a patient and then provides a fan speed control signal to automatically adjust a fan speed to keep the patient comfortable, for example to keep the temperature constant or within an allowed range. In this way, no input is needed from the patient, and the automated control allows clinicians to concentrate or more clinically important tasks.

In one example, the non-contact system 20 is a thermal camera integrated into the bore. Thermal camera modules are available that can measure temperature changes with a precision below 0.05K at frame rates faster than 5Hz. Indeed similar infrared cameras are known which are suitable for use in MR bores, such as the Philips VitalEye (Trade Marks) camera. This infrared camera is currently used for patient sensing, enabling generation of a respiratory signal without any interaction from the operator.

One approach is to measure the temperature of the face at multiple locations and to generate an average over the face area. Face recognition software may be applied to segment the face and thereby enable processing of a set of temperature measurements at a set of defined facial locations.

An initial temperature measurement is for example made within approximately 1 minute of the patient entering the MR bore, thereby to provide a temperature reference $T_{ref}$. If the patient is to be scanned with a head coil, the camera view to the face will be limited. In such a case, the temperature of a particular point on the face or set of points (e.g. a cheek or both cheeks) may be measured with a laser infrared thermometer built into the head coil.

The estimated patient temperature is used to adjust a fan speed.

A first approach is to adjust the fan speed based on the estimated temperature only. The fan speed is adjusted based on the current estimate temperature T using a proportional-integral (PI) or even proportional-integral-differential (PID) temperature controller.

The differential term may however be negligible because no sudden changes of the surface temperature of the face are expected. It is still included in the following function to control fan speed s for the sake of completeness:

$$s(t) = k_p(T - T_{ref}) + k_i \int_{t-\Delta t}^{t}(T - T_{ref})dt + k_d \frac{dT}{dt} \quad \text{Eq. 1}$$

The fan speed setting is thus based on (i) the difference between the estimated temperature and the reference temperature, (ii) the integral over time of this temperature difference and (iii) the differential over time of this temperature difference.

In this equation, $k_p$, $k_i$, and $k_d$ are weighting parameters for the different controller terms and $\Delta t$ is the integration time of the integral component. These parameters can be tuned empirically to allow for smooth temperature control without overshoots or oscillation, or they may be derived by automatically by PID tuning software.

A second approach is to adjust the fan speed based on the estimated temperature and further known relevant physical quantities. In this way, more refined models for heat exchange of the patient with the environment may be used to control fan speed. One example is to include a term for the known specific absorption rate of a particular scan into Eq.1 , which directly changes fan speed if a high SAR scan is applied:

$$s(t) = k_p(T - T_{ref}) + k_i \int_{t-\Delta t}^{t}(T - T_{ref})dt + k_d \frac{dT}{dt} + k_{SAR} SAR(t)\, m \qquad \text{Eq. 2}$$

There is an additional term based on the product of the specific absorption rate of a particular scan SAR(t) in units [W/kg] and the mass m of the patient.

Other control algorithms may be used, for example based on only the temperature difference or the absolute temperature (i.e. proportional control), with threshold levels for the temperature difference corresponding to different fan speed setting and with hysteresis to create stability.

A further option is to enable patient input. Figure 2 shows a microphone and speaker system 24. The microphone and speaker system 24 are part of a voice command system for receiving voice commands from the patient, and the controller 22 is then configured to adapt the fan speed control signal in dependence on the voice commands. These voice commands allow the patient to provide input, which may for example be adapted to alter the automated control, for example by changing the reference temperature.

By way of example, it may not be convenient for the patient to maintain the initial temperature $T_{ref}$ but rather to set a lower temperature (e.g. due to nervousness making the patient feel uncomfortably warm) or a higher temperature (e.g. due to reduced physical action making the patient feel cold). Therefore, a voice input function of the MR system may ask after few minutes of scanning whether the temperature is convenient or should be adjusted.

Closed questions and natural language processing may be used to adjust the reference temperature $T_{ref}$. . For example, closed questions may be provided as audio output such as "Is the current temperature convenient?". If the answer is "no": "Should the temperature be decreased?". If answer is "no": "Should the temperature be increased?".

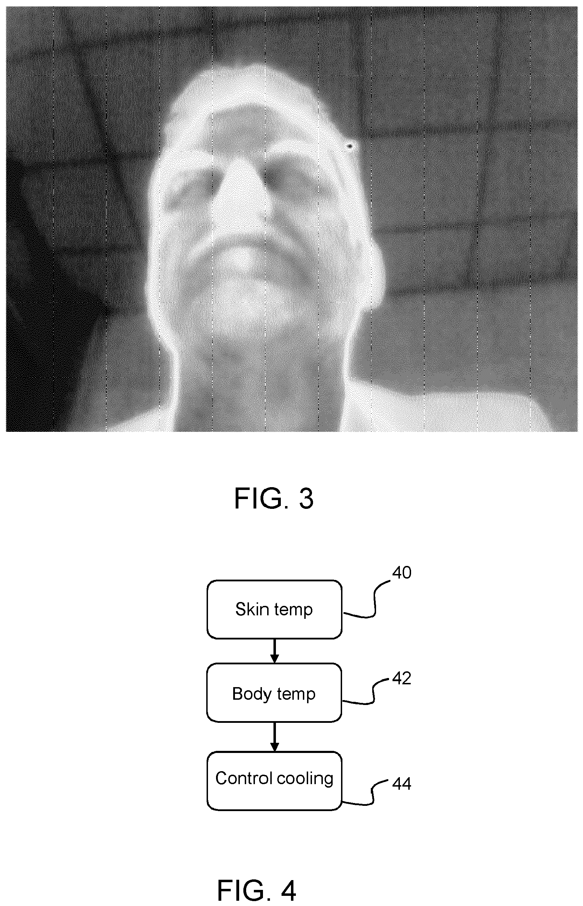

Figure 3 shows a thermal camera image showing how very small temperature differences at different facial locations can be captured (by the greyscale of the image).

Figure 4 shows a method for controlling the temperature of a patient during an MR scan, comprising:

in step 40, performing a skin temperature measurement of the face using a non-contact system;
in step 42, estimating a patient temperature based on the skin temperature measurement; and
in step 44 controlling a fan speed in dependence on the estimated patient temperature.

The example above is based on controlling a ventilator fan speed, and indeed existing MR scanners have such ventilator fans. Thus, the system is for passive cooling control. The invention may be extended to include control of active heating and/or active cooling. Active heating is typically not required since the MR scanner itself generates heat. However, an active cooling system may be controlled, such that a set temperature of the cooling system as well as or instead of a fan speed setting may be controlled.

An example of a possible active cooling system is to provide air cooling via tubing built into into the surface coils wrapped around the patient. These may release cold air directly at the patient's clothes. Alternatively, the fan may include a cooling system such that cooled air is delivered generally to the MR bore instead of a flow of air at the prevailing ambient temperature.

As discussed above, embodiments make use of a controller. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

In various implementations, a processor or controller may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor or controller.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems. (optional)

If the term "adapted to" is used in the claims or description, it is noted the term "adapted to" is intended to be equivalent to the term "configured to".

Any reference signs in the claims should not be construed as limiting the scope.

**Claims**

1. A system for controlling the temperature of a patient during an MR scan, comprising:

   a non-contact system (20) for performing a skin temperature measurement of the face; and
   a controller (22), configured to:

   estimate an overall patient temperature based on the skin temperature measurement; and
   provide a cooling system control signal for controlling a cooling system in dependence on the estimated patient temperature, for cooling the patient.

2. The system of claim 1, wherein the cooling system control signal is a fan speed control signal for controlling a fan speed, for providing ventilation to the patient.

3. The temperature control system of claim 1 or 2, wherein the controller (22) is configured to determine an average temperature for a plurality of locations of the face.

4. The temperature control system of any one of claims 1 to 3, wherein the controller (22) is configured to derive a reference temperature within a first time period of the patient entering an MR scanner bore.

5. The temperature control system of claim 4, wherein the first time period is in the range 30 seconds to 5 minutes.

6. The temperature control system of any one of claims 1 to 5, wherein the non-contact system (20) comprises an infrared camera system or a laser infrared thermometer.

7. The temperature control system of any one of claims 1 to 6, wherein the controller (22) is configured to to provide a fan speed control signal for controlling a fan speed to maintain a constant estimated temperature using proportional-integral control or proportional-integral-differential control.

8. The temperature control system of claim 7, wherein the proportional-integral control or proportional-integral-differential control uses:

   only the estimated temperature as input parameter; or
   the estimated temperature and thermal absorption characteristics of the MR scan as inputs.

9. The temperature control system of any one of claims 1 to 8, further comprising a voice command system (24) for receiving voice commands from the patient, and the controller (22) is configured to adapt the fan speed control signal in dependence on the voice commands.

10. A magnetic resonance imaging system, comprising:

    an MR bore;
    a cooling system for cooling a patient in the MR bore; and
    the temperature control system (20,22) of any one of claims 1 to 9 for controlling the cooling system (10).

11. A computer implemented method for controlling the temperature of a patient during an MR scan, comprising:

    (40) performing a skin temperature measurement of the face using a non-contact system;
    (42) estimating an overall patient temperature based on the skin temperature measurement; and
    (44) controlling a cooling system in dependence on the estimated patient temperature for cooling the patient.

12. The method of claim 11, comprising:
    performing the skin temperature measurement of the face using an infrared camera system or a laser infrared thermometer.

13. The method of claim 11 or 12 comprising controlling a fan speed to maintain a constant estimated temperature using proportional-integral control or proportional-integral-differential PID control.

**14.** A computer program comprising computer program code means which is adapted, when said program is run on a computer, to implement the method of any one of claims 12 to 13.

FIG. 1

FIG. 2

FIG. 3

FIG. 4

Europäisches
Patentamt

European
Patent Office

Office européen
des brevets

# EUROPEAN SEARCH REPORT

Application Number

EP 21 15 7287

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2017/227973 A1 (SCHNETTER VOLKER [DE]) 10 August 2017 (2017-08-10) | 1,2,4,5, 7,8,10, 11,13,14 | INV. G01R33/28 A61B5/01 |
| Y | * paragraphs [0010] - [0015], [0031], [0038], [0039], [0041] - [0043]; figures 1,2 * | 3,6,9,12 | |
| X,D | WO 2016/087376 A1 (KONINKL PHILIPS NV [NL]) 9 June 2016 (2016-06-09) | 1,2,4,5, 7,8,10, 11,13,14 | |
| Y | * claims 1-15; figure 1 * | 1,3,6,9, 12 | |
| Y | JP 5 300602 B2 (MITSUBISHI ELECTRIC CORP) 25 September 2013 (2013-09-25) * paragraphs [0001] - [0003], [0022] - [0024], [0031]; figure 4 * | 1 | |
| Y | US 2016/228005 A1 (BAMMER ROLAND [US] ET AL) 11 August 2016 (2016-08-11) * paragraph [0037]; claim 12; figure 1 * | 1 | |
| Y | WO 2015/117084 A1 (UNIV LELAND STANFORD JUNIOR [US]) 6 August 2015 (2015-08-06) * page 4, line 13 - line 17; figure 1 * | 1 | TECHNICAL FIELDS SEARCHED (IPC) G01R A61B |
| Y | CN 111 401 349 A (GUANGZHOU TUPU NETWORK TECH CO LTD) 10 July 2020 (2020-07-10) * claims 1-3, 5; figures 2,4-7 * | 3 | |
| Y | CN 210 624 837 U (CHIPINTELLI TECH CO LTD) 26 May 2020 (2020-05-26) * claims 1-6; figures 1,2 * | 9 | |
| Y | US 2018/271396 A1 (WATTS RAYMOND C [US] ET AL) 27 September 2018 (2018-09-27) * paragraphs [0039] - [0044], [0053] - [0055]; figures 1-4, 18-22 * | 6,12 | |

The present search report has been drawn up for all claims

| Place of search | Date of completion of the search | Examiner |
|---|---|---|
| Munich | 21 July 2021 | Faber-Jurk, Sonja |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons
........................................................................
& : member of the same patent family, corresponding
document

EPO FORM 1503 03.82 (P04C01)

**EP 4 043 900 A1**

## ANNEX TO THE EUROPEAN SEARCH REPORT
## ON EUROPEAN PATENT APPLICATION NO.

EP 21 15 7287

This annex lists the patent family members relating to the patent documents cited in the above-mentioned European search report.
The members are as contained in the European Patent Office EDP file on
The European Patent Office is in no way liable for these particulars which are merely given for the purpose of information.

21-07-2021

| Patent document cited in search report | | Publication date | Patent family member(s) | | Publication date |
|---|---|---|---|---|---|
| US 2017227973 | A1 | 10-08-2017 | DE 102016201908 | A1 | 10-08-2017 |
| | | | US 2017227973 | A1 | 10-08-2017 |
| WO 2016087376 | A1 | 09-06-2016 | CN 107110923 | A | 29-08-2017 |
| | | | EP 3227699 | A1 | 11-10-2017 |
| | | | JP 6448794 | B2 | 09-01-2019 |
| | | | JP 2017536199 | A | 07-12-2017 |
| | | | RU 2676538 | C1 | 09-01-2019 |
| | | | US 2017269176 | A1 | 21-09-2017 |
| | | | WO 2016087376 | A1 | 09-06-2016 |
| JP 5300602 | B2 | 25-09-2013 | JP 5300602 | B2 | 25-09-2013 |
| | | | JP 2010133692 | A | 17-06-2010 |
| US 2016228005 | A1 | 11-08-2016 | CN 105555189 | A | 04-05-2016 |
| | | | DE 112014004250 | T5 | 23-06-2016 |
| | | | JP 6499662 | B2 | 10-04-2019 |
| | | | JP 2016530054 | A | 29-09-2016 |
| | | | US 2016228005 | A1 | 11-08-2016 |
| | | | WO 2015042138 | A1 | 26-03-2015 |
| WO 2015117084 | A1 | 06-08-2015 | CN 106456045 | A | 22-02-2017 |
| | | | DE 112015000614 | T5 | 27-10-2016 |
| | | | JP 6426201 | B2 | 21-11-2018 |
| | | | JP 2017505697 | A | 23-02-2017 |
| | | | US 2016331239 | A1 | 17-11-2016 |
| | | | WO 2015117084 | A1 | 06-08-2015 |
| CN 111401349 | A | 10-07-2020 | NONE | | |
| CN 210624837 | U | 26-05-2020 | NONE | | |
| US 2018271396 | A1 | 27-09-2018 | EP 3212080 | A2 | 06-09-2017 |
| | | | JP 6717844 | B2 | 08-07-2020 |
| | | | JP 2017533073 | A | 09-11-2017 |
| | | | US 2018271396 | A1 | 27-09-2018 |
| | | | WO 2016069967 | A2 | 06-05-2016 |

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016087376 A